# FASCICULE DE BREVET EUROPEEN

(11) **EP 3 518 916 B1**
(45) Date de publication et mention de la délivrance du brevet: **07.07.2021**
(21) Numéro de dépôt: 17777046.8
(22) Date de dépôt: 28.09.2017
(51) Int. Cl.: A61K 31/192, A61K 47/18, A61K 9/00, A61P 1/16

(54) **COMPOSITION COMPRENANT AU MOINS UN SEL PHARMACEUTIQUEMENT ACCEPTABLE D'ELAFIBRANOR SOLUBLE EN MILIEUX AQUEUX PRESENTANT UNE ABSORPTION INTESTINALE AMELIOREE**
ZUSAMMENSETZUNG MIT MINDESTENS EINEM WASSERLÖSLICHEN PHARMAZEUTISCH AKZEPTABLEN SALZ VON ELAFIBRANOR MIT VERBESSERTER INTESTINALER ABSORPTION
COMPOSITION COMPRISING AT LEAST ONE WATER-SOLUBLE PHARMACEUTICALLY ACCEPTABLE SALT OF ELAFIBRANOR HAVING IMPROVED INTESTINAL ABSORPTION

(30) Priorité: 30.09.2016 FR 1659438
(43) Date de publication de la demande: 07.08.2019
(73) Titulaire: Nashpharm, 06270 Villeneuve Loubet (FR)
(72) Inventeur: LARUELLE, Claude, 06270 Villeneuve Loubet (FR); BONNAFOUS, Ludovic, 06370 Mouans Sartoux (FR)
(74) Mandataire: Brizio Delaporte, Allison
(86) Numéro de dépôt international: PCT/EP2017/074701
(87) Numéro de publication internationale: WO 2018/060372

(56) Documents cités:
- EP-A1- 1 525 177
- EP-A1- 2 641 596
- WO-A1-2016/154258
- US-A1- 2005 148 594
- PIYUSH PATEL ET AL: "Comparison of efficacy and safety of choline fenofibrate (fenofibric acid) to micronized fenofibrate in patients of mixed dyslipidemia: A randomized, open-label, multicenter clinical trial in Indian population", INDIAN JOURNAL OF ENDOCRINOLOGY AND METABOLISM, vol. 20, no. 1, 1 janvier 2016 (2016-01-01), page 67, XP055331589, ISSN: 2230-8210, DOI: 10.4103/2230-8210.172243

## Description

### DOMAINE TECHNIQUE

La présente invention concerne des médicaments dérivés d'élafibranor.

L'invention concerne notamment une composition soluble en milieux aqueux présentant une absorption intestinale améliorée. Elle porte sur l'usage de sels pharmaceutiquement acceptables d'élafibranor (GFT505), utilisables dans des compositions pharmaceutiques.

L'invention concerne plus particulièrement l'utilisation de sels d'élafibranor en vue d'améliorer la stabilité et la solubilité comparativement à l'élafibranor sous sa forme base. Ces sels permettent d'établir des formulations pharmaceutiques sous diverses formes avantageuses comme les injections intraveineuses ou des formulations par voie entérale présentant une absorption plus rapide, moins variable et par conséquent une biodisponibilité meilleure.

### ETAT DE LA TECHNIQUE

L'élafibranor, également cité sous son nom de code GFT505, est une molécule expérimentale de la société Genfit développée initialement pour le traitement des maladies métaboliques incluant le diabète, la résistance à l'insuline, la dyslipidémie. Sa cible thérapeutique actuelle est le traitement des maladies hépatiques, en particulier la stéato-hépatite non alcoolique (NASH).

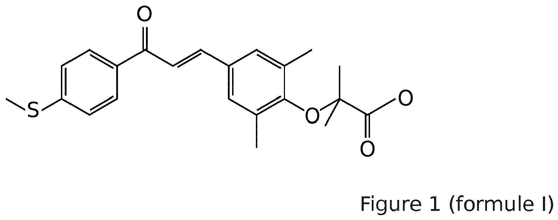

Son nom chimique est acide 2-[2,6 dimethyl-4-[3-[4-(methylthio)phenyl]-3-oxo-1(E)-propenyl]phenoxyl]-2-methylpropanoïque, de formule chimique C₂₂H₂₄O₄S et de poids moléculaire de 384,489 g/mol. Sa structure chimique de formule I est donnée dans la figure 1

Selon le brevet EP 1525177 B1 de Genfit qui décrit l'utilisation, la préparation de molécules de la famille des 1,3-diphénylprop-2-en-1-one, l'élafibranor, appelé composé 29 dans ce document, est identifié par un spectre RMN (1H DMSO) avec les caractéristiques suivantes (δ ppm): 1.39 (s, 6H), 2.22 (s, 6H), 2.57 (s, 3H), 7.40 (d, J = 8.55Hz, 2H), 7.57 (s, 2H), 7.62 (d, J = 15.5Hz, 1H), 7.83 (d, J = 15.5Hz, 1H), 8.10 (d, J = 8.55Hz, 2H), 12.97 (s, 1H) SM (ES- MS) : 383.3 (M-1).

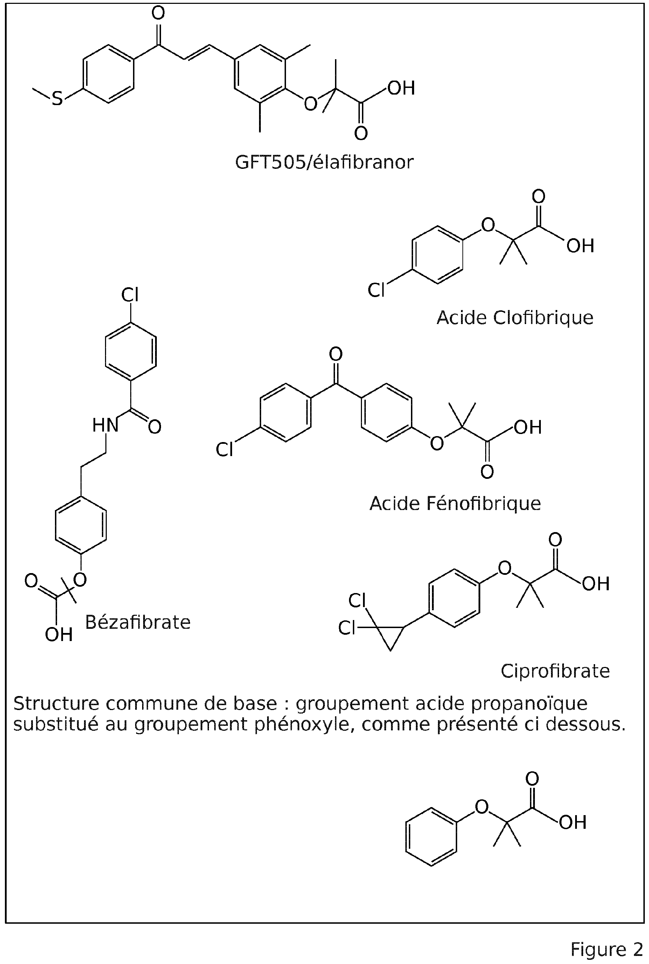

Il n'y a pas d'autres éléments publiquement disponibles apportant de plus amples précisions sur l'identification physico-chimique de cette molécule qui comporte le même groupement acide propanoïque phénoxylé que les molécules de la famille des fibrates (figure 2).

Les molécules appartenant à la famille des fibrates sont connues pour leur faible solubilité aqueuse, que ce soit sous la forme d'ester ou d'acide carboxylique comme l'est l'élafibranor (GFT505). De plus, il est à noter des variabilités notables dans la pharmacocinétique de ces molécules, comme par exemple pour le fénofibrate dont les formulations pharmaceutiques ont fait l'objet d'apports réguliers de travaux d'innovation galénique pour remédier à ces inconvénients et ainsi améliorer la biodisponibilité.

Les autres informations disponibles sur l'élafibranor concernent essentiellement les propriétés précliniques, cliniques et toxicologiques de cette molécule. L'élafibranor y est en effet identifié comme étant un co-activateur des récepteurs nucléaires PPARα/δ. Tous les essais cliniques ont montré un très bon profil de tolérance de cette molécule, conforté notamment par des études toxicologiques à fortes doses chez l'animal incluant des études de carcinogénicité.

L'élafibranor (GFT05) présente en outre des effets bénéfiques sur la stéato-hépatite non alcoolique NASH avec l'amélioration des marqueurs biochimiques de dysfonctionnement hépatique, notamment les enzymes hépatiques : ALAT, ASAT, γGT, et ALP7.

Actuellement, il n'y a pas de description approfondie des données physico-chimiques de l'élafibranor, qu'il s'agisse de la molécule chimique seule ou en tant que molécule active dans une composition pharmaceutique. Aucun sel physiologiquement acceptable n'est décrit dans des brevets ou publications scientifiques.

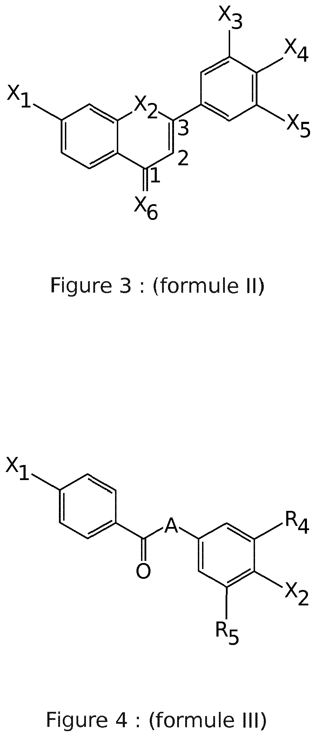

L'élafibranor (GFT505) est décrit depuis 2003 dans plusieurs brevets de la société Genfit qui couvrent toute application thérapeutique et, depuis 2009, sur une nouvelle application thérapeutique spécifique, en particulier pour le traitement de la stéato-hépatite non alcoolique NASH.
Les brevets EP1525177 et US7943661 portent sur une nouvelle famille de dérivés des chalcones. Ils décrivent le procédé de préparation et l'utilisation de dérivés de 1,3-diphénylprop-2-èn-1-one substitués de formule II ci-après (figure 3), dont fait partie la molécule élafibranor (composé 29 décrit dans la description, revendication 25), pour toute application thérapeutique, sans limitation à une maladie précise.

La seconde famille de brevets EP2504005, US8772342 et US9221751 porte sur des composés pour utilisation dans une méthode de traitement d'un trouble hépatique choisi dans le groupe constitué par une fibrose hépatique ou une stéatose hépatique. Notamment, la revendication 7 porte sur la molécule élafibranor pour une utilisation dans le traitement d'une fibrose hépatique ou d'une stéatose hépatique. Les revendications 9 et 10 portent sur une composition pharmaceutique comprenant un composé de formule III ci-dessous (figure 4) dans une méthode de traitement d'un trouble hépatique choisi dans le groupe constitué par une fibrose hépatique ou une stéatose hépatique.

Le brevet EP2504005B1 a fait l'objet d'une demande divisionnaire EP2641596A1 concernant les composés revendiqués dans le brevet EP2504005B1, mais utilisés cette fois-ci uniquement dans le cadre spécifique des maladies : cirrhose du foie, maladies liées à l'alcool, maladies hépatiques à médiation immunitaire.

D'autres brevets traitant de l'élafibranor sont à noter. Le brevet US7566737B porte sur une composition pharmaceutique comprenant une association entre un dérivé de 1,3-diphénylprop-2-èn-1-one substitué de formule II, y compris la molécule élafibranor, et un autre ingrédient ayant une activité thérapeutique.

Le brevet US8895619B traite d'une méthode de traitement d'une fibrose hépatique par l'administration de la molécule Elafibranor (revendications 1-7, 10-11) et notamment pour traiter la cirrhose (revendications 8-9).

La demande US2016/0051501 porte sur une méthode de traitement d'une maladie du foie virale ou liée à l'alcool ou immunitaire par un composé de formule III.

L'élafibranor n'est pas cité dans d'autres brevets. Seuls des résultats d'études apparaissent dans plusieurs articles dont les premiers sont publiés dès 2007 (Fruchart, Am J Cardiol 2007; 100 [suppl]:41N-46N ; en 2013 : Fruchart Cardiovascular Diabetology 2013, 12:82).

Le poster « The hepatic and extra-hepatic profile of resolution of steatohepatitis induced by GFT-505 (élafibranor)" de Sanyal AJ et al., traite des résultats d'une étude de phase 2b (Golden505) mettant en avant une dose journalière de 80 ou 120 mg en élafibranor administrée sur 270 patients NASH (3 groupes dont des diabétiques et non diabétiques). Il n'y a aucune précision sur la composition pharmaceutique des gélules dosées à 40 mg utilisées pour cette étude ni sur les caractéristiques physico-chimiques de l'élafibranor ou le rationnel concernant l'administration avant le petit déjeuner.

Les paramètres pharmacocinétiques dont le métabolisme ne sont pas publiquement disponibles pour l'élafibranor en dépit des études de phase 1 qui ont été réalisées. En 2012, dans l'étude de recherches de doses « *Comparative Bioavailability - Gender Effect* - *Single and Multiple Ascending Dose Safety and Pharmacokinetic Study of GFT505",* des modifications ont été apportées par la société Genfit dans les formulations d'élafibranor. Une étude de la biodisponibilité relative entre nouvelles et anciennes formulations a été menée, sur une gamme de dose atteignant 300 mg. Il n'y a pas de publication de résultats ni d'informations qui viennent justifier et étayer les raisons de ces travaux de formulation.

L'élafibranor telle que décrite dans les brevets de Genfit est mise dans des formes galéniques particulières. Notamment, l'élafibranor ne se trouve pas sous forme d'injections intraveineuses ou dans des formulations efficaces par voie entérale.

De façon surprenante, il a été trouvé que la mise en oeuvre de sels pharmaceutiquement acceptables spécifiques d'Elafibranor améliore la stabilité et la solubilité de ce dernier, permettant ainsi de nouvelle forme galénique et une meilleure biodisponibilité.

### BRÈVE DESCRIPTION DES FIGURES

Les buts, objets, ainsi que les caractéristiques et avantages de l'invention ressortiront mieux de la description détaillée d'un mode de réalisation de cette dernière qui est illustré par les figures d'accompagnement suivantes dans lesquelles :
Figure 1 : Formule chimique de l'Elafibranor.
Figure 2 : Formule chimique de fibrates et du groupement chimique commun des fibrates et de l'élafibranor.
Figure 3: Formule chimique dérivé du 1,3-diphénylprop-2-èn-1-one substitué comprenant l'Elafibranor.
Figure 4 : Formule générale d'un composé de la demande de brevet EP2504005 comprenant l'Elafibranor.
Figure 5 : Schéma de synthèse de l'élafibranor (GFT505)
Figure 6A: Spectre RNM 1H élafibranor (GFT505)
Figure 6B : UPLCMS élafibranor (GFT505)
Figure 6C : Spectre UV élafibranor (GFT505)
Figure 7A: Spectre RMN 1H du sel de choline d'élafibranor (GFT505)
Figure 7B: Spectres UV/IPLCMS du sel de choline d'élafibranor (GFT505)

### EXPOSE DE L'INVENTION

Avant d'entamer une revue détaillée de modes de réalisation de l'invention, sont énoncées ci-après des caractéristiques optionnelles qui peuvent éventuellement être utilisées en association ou alternativement.

L'invention concerne une composition comprenant comme principe actif un sel pharmaceutiquement acceptable d'élafibranor caractérisée en ce que le sel pharmaceutiquement acceptable d'élafibranor est un sel de choline.

Avantageusement, la composition est sous une forme adaptée pour une administration par voie entérale.

Avantageusement, la composition est sous une forme adaptée pour une administration par voie parentérale.

Les modes d'administration par voie parentérale permettent une absorption rapide et une biodisponibilité optimale.

Avantageusement, la composition est sous une forme adaptée pour une administration par voie intraveineuse.

Avantageusement, le sel d'élafibranor est micronisé ou de structure amorphe.

Avantageusement, le sel d'élafibranor est sous forme d'une poudre pour préparation injectable soluble.

Avantageusement, la composition est sous une forme adapté pour une administration par voie sous -cutanée.

Avantageusement, la composition comporte au moins un excipient choisi parmi les liants, les agents désintégrants, les diluants, les lubrifiants, les agents tensioactifs, les agents tampons, les agents d'écoulement, les colorants, les arômes, les édulcorants, les solvants ou agents conservateurs.

Avantageusement, la composition comporte plus de 50% des particules de taille inférieure ou égale à 10 µm et l'ensemble des particules de taille inférieure à 20 µm.

Avantageusement, le sel pharmaceutiquement acceptable d'élafibranor est configuré pour avoir une solubilité au moins égale à 10 mg/ml en milieu physiologique NaCl 0.9 %.

Avantageusement, le sel d'élafibranor est configuré pour présenter un profil de dissolution en milieux FaSSIF et FeSSIF simulés supérieur à 90 % après 30 minutes.

Avantageusement, le sel pharmaceutiquement acceptable d'élafibranor (GFT505) est configuré pour être photostable.

On entend par photostable la capacité à être moins sensible à la lumière.

Avantageusement, la composition est destinée à être utilisée dans le traitement des maladies hépatiques.

Avantageusement, la maladie hépatique consiste en la stéatose hépatique non alcoolique (NAFLD).

Avantageusement, la composition est destinée à être utilisée dans le traitement des maladies hépatiques caractérisé en ce que la maladie hépatique consiste en la stéato-hépatite non alcoolique (NASH).

Avantageusement, la maladie hépatique consiste en la fibrose hépatique.

Avantageusement, la maladie hépatique consiste en la cirrhose.

Avantageusement, la maladie hépatique consiste en des maladies auto-immunes hépatiques.

Avantageusement, le mode d'administration consiste en une administration par voie orale.

Avantageusement, le mode d'administration consiste en une administration par voie sous cutanée.

Avantageusement, la forme médicamenteuse consiste en une poudre pour suspension orale.

Le sel pharmaceutiquement acceptable d'élafibranor présente l'avantage d'avoir une meilleure solubilité dans l'eau comparativement à la forme base.

Avantageusement, la forme médicamenteuse consiste en la forme d'une solution injectable, d'un comprimé, d'un comprimé dispersible, d'un comprimé orodispersible, d'une gélule, d'un comprimé soluble, d'un lyophilisat, d'un comprimé effervescent, d'un comprimé à croquer, d'un comprimé à libération prolongée, d'un sachet.

Avantageusement, le profil de dissolution en milieux FaSSIF et FeSSIF simulés du sel pharmaceutiquement acceptable d'élafibranor présente un pourcentage de dissolution supérieur à 90 % après 30 minutes.

Avantageusement, la forme médicamenteuse comporte des particules d'élafibranor, avec au moins 50% des particules étant de tailles inférieures à 10 µm.

Avantageusement, la composition comporte au moins un excipient physiologiquement acceptable, notamment au moins un parmi les liants, les agents désintégrants, les diluants, les lubrifiants, les agents tensioactifs, les agents tampons, les agents d'écoulement, les colorants, les arômes, les édulcorants, les solvants ou agents conservateurs.

L'invention concerne une utilisation d'une composition comprenant comme principe actif au moins un sel de choline d'élafibranor (GFT505) pour l'obtention d'un médicament destiné à une utilisation dans traitement ou la prévention de maladies, en particulier en hépatopathie incluant, de manière non exhaustive, la stéatose hépatique non alcoolique (NAFLD), la stéato-hépatite non alcoolique (NASH), la fibrose hépatique, la cirrhose, les maladies auto-immunes hépatiques.

Dans un autre aspect, l'invention concerne la préparation d'un sel de choline d'élafibranor (GFT505) démontrant des propriétés physico-chimiques plus avantageuses que la forme base d'élafibranor, en particulier sur le plan de la solubilité et/ou de la stabilité.

### DESCRIPTION DETAILLEE

La présente invention se rapporte à l'utilisation d'un sel pharmaceutiquement acceptable d'élafibranor ainsi que l'un quelconque de ses dérivés, sous l'une de ses formes cristallines éventuellement polymorphes, ou bien selon une structure cristalline amorphe, dans la préparation d'un médicament pour le traitement ou la prévention des maladies, en particulier les maladies du foie comme les stéatoses hépatiques non alcoolique (NASH et NAFLD), les fibroses, les cirrhoses ou cancers, les maladies auto-immunes.

L'invention concerne également l'utilisation d'un sel pharmaceutiquement acceptable de l'acide 2-[2,6 dimethyl-4-[3-[4-(methylthio)phenyl]-3-oxo-1(E)-propenyl]phenoxyl]-2-methylpropanoïque, de formule chimique C₂₂H₂₄O₄S (figure 1), pouvant être utilisés dans une composition pharmaceutique pour prévenir ou traiter les maladies, en particulier les maladies du foie.

Par "sel pharmaceutiquement acceptable", on entend un sel de choline.

Le sel pharmaceutiquement acceptable est par exemple sous l'une de ses formes cristallines éventuellement polymorphes, ou bien selon une structure cristalline amorphe.

Le sel d'élafibranor est également dénommé sel d'élafibranorate.

La composition pharmaceutique de l'invention peut être administrée par voie entérale, par voie parentérale, topique ou sous cutanée. Selon un mode d'administration, la composition est administrée par voie entérale, tels que, par exemple, un comprimé, une gélule, une capsule molle, un lyophylisat, un comprimé dispersible, orodispersible, effervescent ou soluble, une solution orale, une poudre pour suspension orale.

Selon un mode préféré d'administration, la composition est administrée par voie intraveineuse, par voie sous cutanée, sous la forme par exemple d'une solution injectable, d'une poudre pour solution injectable.

Les formulations destinées à être administrées par la voie intraveineuse ou orale contiennent avantageusement le sel d'élafibranor micronisé ou de structure amorphe afin d'optimiser la solubilité aqueuse et la dissolution entérique le cas échéant. La distribution de la taille des particules du sel d'élafibranor qui présentent une structure cristalline est caractérisée en ce sens que plus de 50% des particules sont inférieures ou égales à 10 µm, et toutes les particules ont une taille inférieure à 20 µm.

Les particules sont mesurées à l'aide d'un granulomètre laser type Malvern ou équivalent, et une méthode validée par voie humide (mouillage avec un tensio-actif) est préférée.

L'une des compositions pharmaceutiques préférées de l'invention est une poudre pour préparation injectable soluble et stable en conditions normales de température et d'humidité, soit 25°C/60%HR (conditions ICH).

Les exemples suivants sont donnés pour illustrer l'invention et ne constituent en aucune manière une limitation de celle-ci.

### EXEMPLES

### EXEMPLE 1: Synthèse et caractérisation de l'élafibranor (GFT505)

La demanderesse a décidé de préparer des échantillons d'élafibranor pour évaluer la faisabilité des étapes de la synthèse de cette molécule et caractériser les propriétés physico-chimiques du produit obtenu. Le mode opératoire s'inspire des informations décrites dans le brevet EP 1525177 B1 pour la synthèse du composé 29. Les étapes sont reproduites à l'identique.

### Protocole expérimental

Le composé est synthétisé à partir de 1-[4-méthylthiophényl]-3-[3,5-diméthyl-tertiobutyloxycarbonyldiméthylméthyloxyphényl]prop-2-èn-1-one.

### Etape 1 : 1-[4-méthylthiophényl]-(E)-3-[3,5-diméthyl-4-hydroxyphényl]prop-2-èn-1-one (intermédiaire 1)

La 4-méthylacétophénone (20 g, 0,12 mol, 1 eq) et le 3,5-diméthyl-4-hydroxybenzaldehyde (18 g, 0,12 mol, 1 eq) sont solubilisés dans 300mL de HCl 4N dans le dioxane. Le milieu réactionnel est agité 30 h puis les solvants sont évaporés. Purification par recristallisation à chaud dans 70 mL d'isopropanol et 12mL d'eau : 33 g (solide jaune, rendement : 92 %).
Formule brute : C₁₈H₁₈O₂S
ESI-MS m/z=299,18 [M+H]+
RMN ¹H DMSO-d6 δ ppm : 2.18 (s, 6H), 2.53 (s, 3H), 7.36 (d, J=8,5Hz, 2H), 7.47 (s, 2H), 7.57 (d, J= 15,5Hz, 1H), 7,69 (d, J= 15,5Hz, 1H), 8,05 (d, J= 8,5Hz, 2H), 8,93 (s, 1H)

### Etape 2 : 1-[4-méthylthiophényl]-(E)-3-[3,5-diméthyl-4-tertiobutylcarbonyldiméthylméthyloxyhényl]prop-2-èn-1 -one (intermédiaire 2)

Le carbonate de césium (87 g, 0,134 mol, 4 eq) et l'iodure de tétrabutylammonium (12 g, 0,033 mol, 0,5 eq) sont ajoutés à une solution de l'intermédiaire 1 (20 g, 0,067 mol, 1 eq) dans 50mL d'un mélange DMSO/eau (3/2). Le milieu réactionnel est agité 30min à 80°C et le bromoisobutyrate de tertiobutyle (30 g, 0,134 mol, 2 eq) est ajouté. Puis 2 ajouts de 2 eq de bromoisobutyrate de tertiobutyle dilué à 50% dans le DMSO sont effectués chacun à 1 heure d'intervalle. Le milieu réactionnel est agité 2 jours à 80°C. Le milieu réactionnel est laissé refroidir à température ambiante puis 1,5L d'eau est ajouté et le produit est extrait avec du dichlorométhane (4 fois). La phase organique est séchée sur une cartouche séparatrice de phase et évaporée à sec. Purification sur gel de silice (cyclohexane/acétate d'éthyle : 95/5 à 80/20) : 18g (solide orange, rendement : 61%)
Formule brute : C₂₆H₃₂O₄S
ESI-MS m/z=441,33 [M+H]+
RMN ¹H DMSO-d6 δ ppm : 1.36 (s, 6H), 2.19 (s, 6H), 2.48 (broad peak, H2O + 9H), 2.54 (s, 3H), 7.38 (d, J=8,4Hz, 2H), 7.55 (s, 2H), 7.59 (d, J= 15,6Hz, 1H), 7.80 (d, J= 15,6Hz, 1H), 8.07 (d, J= 8,4Hz, 2H)

### Etape 3 : 1-[4-méthylthiophényl]-(E)-3-[3,5-diméthyl-4-carboxydiméthylméthyloxyhényl]prop-2-èn-1 -one

L'intermédiaire 2 (25 g, 0,057 mol, 1 eq) est solubilisé dans 50 mL de dichloromethane et 22 mL d'acide trifluoroacétique (5 eq, 0,284 mol) est ajouté doucement. Le milieu réactionnel est agité 3h30 à température ambiante puis les solvants sont évaporés à sec. Purification sur gel de silice (dichlorométhane/Méthanol : 100/0 -> 95/5) : 13 g (solide jaune, rendement 60%).
Formule brute : C₂₂H₂₄O₄S
ESI-MS m/z=385,25 [M+H]+
RMN ¹H DMSO-d6 δ ppm : 1.36 (s, 6H), 2.19 (s, 6H), 2.54 (s, 3H), 7.37 (d, J=8,6Hz, 2H), 7.55 (s, 2H), 7.59 (d, J= 15,6Hz, 1H), 7.80 (d, J= 15,6Hz, 1H), 8.07 (d, J= 8,6Hz, 2H), 12.94 (s, 1H)

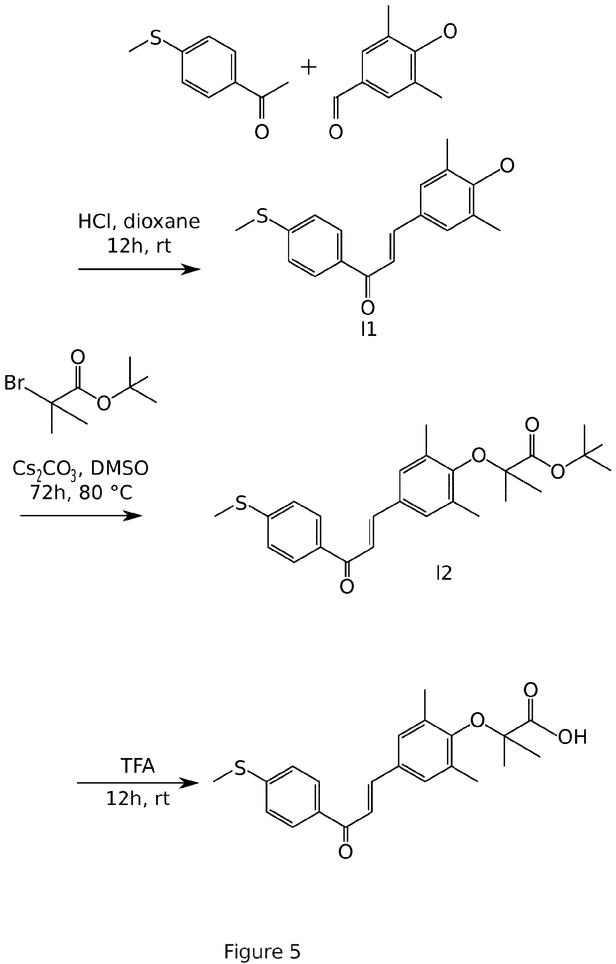

Le schéma réactionnel est donné dans la figure 5.

### Résultats

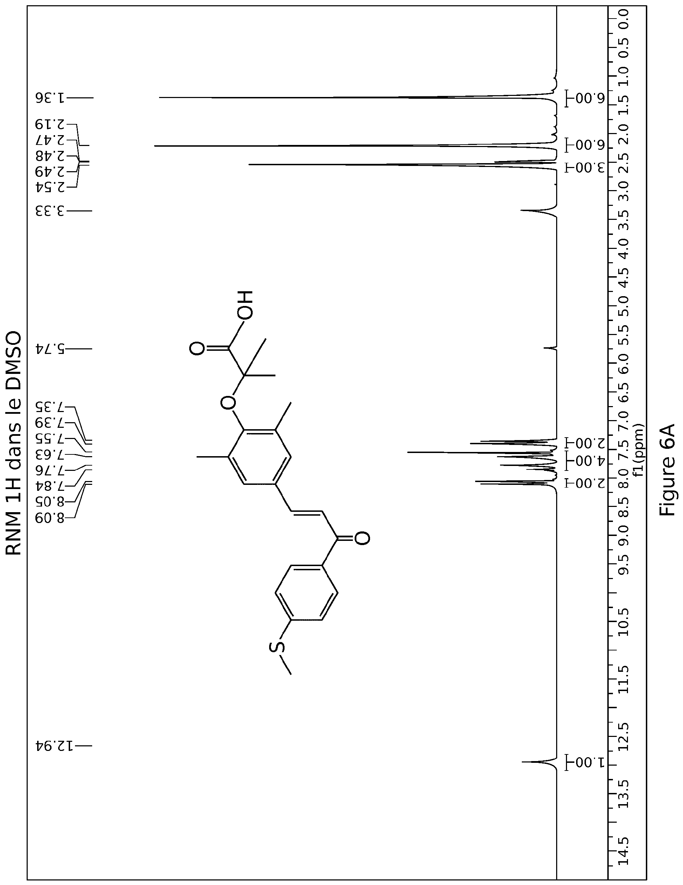

Les données d'analyses des 10,1 g du lot obtenu (EM0274L2) sont résumées ci-dessous :
- Masse moléculaire : 384,5 (masse exacte : 384,1) ;
- Spectre RMN 1H : Conforme à la structure, cf spectre en figure 6A ci-après.
- LCMS : TR=1,42mn, m/z: 385,00 = [M+H]+;
- Pureté : >98% (1H RMN et LCMS) ;
- Point de fusion : 144-145°C.

L'aspect du produit est une poudre solide jaune amorphe. Le produit présente une absorption notable dans le proche visible avec un Apex à environ 347 nm.

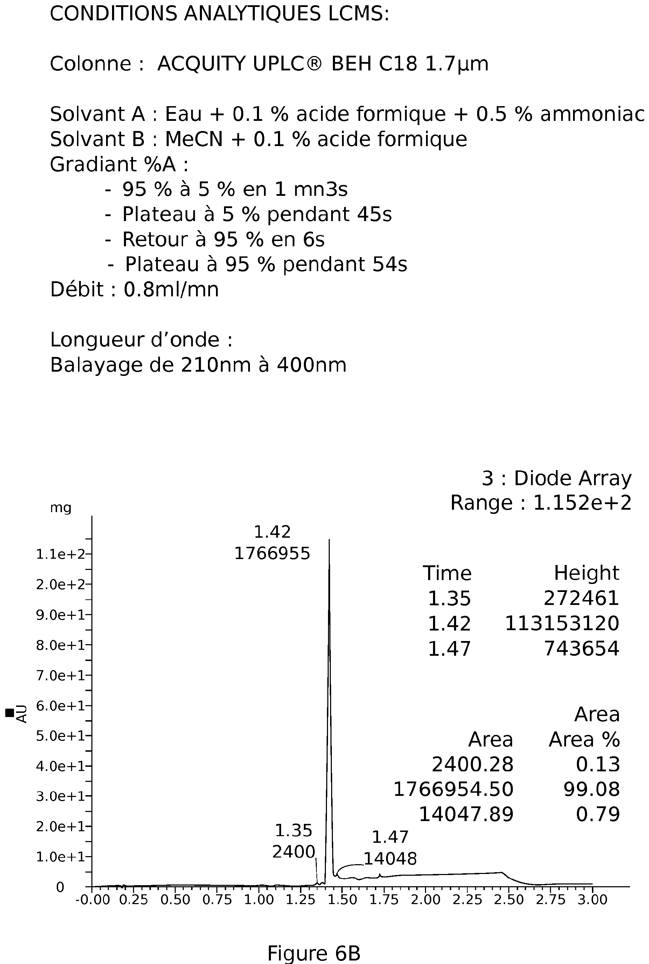

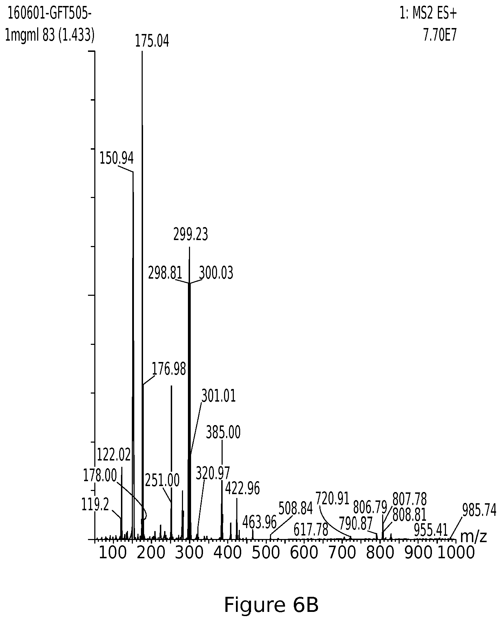

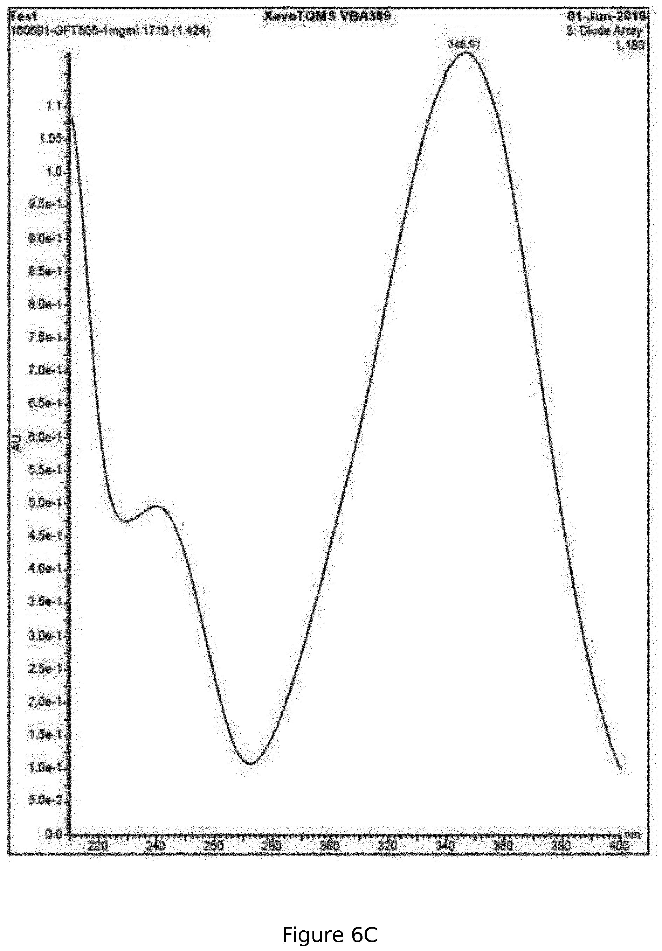

Les informations sont détaillées dans les figures 6.
Le produit obtenu est conforme en termes de pureté chimique et démontre une absorption dans le proche visible qui nécessite que la stabilité chimique à la lumière et la phototoxicité doivent donc être vérifiées.

### EXEMPLE 2: Mesure de solubilité de l'élafibranor (GFT505)

Au cours d'une expérience réalisée pour l'invention, il a été montré que l'élafibranor (GFT505) affichait une structure chimique apparentée à la famille des fibrates (figure 2). L'élafibranor étant un acide carboxylique, la demanderesse a choisi de vérifier la solubilité aqueuse de cette molécule afin de statuer sur la faisabilité de développement de compositions pharmaceutiques en accord avec les attentes des patients, plus efficaces et mieux tolérées par les patients.

### Protocole expérimental

La solubilité thermodynamique de l'élafibranor base est étudiée sur une période de 24 heures et 72 heures dans divers tampons aqueux en présence ou non de tensioactifs. Le lot n°EM0274L2 est utilisé pour ces travaux. Le produit est dissous dans les solvants indiqués dans le tableau 1. Après 24h et 72h d'incubation à température ambiante (22 - 24°C), les solutions sont prélevées puis filtrées sur filtres en polycarbonate 0.2 µm dans des flacons pour analyses LCMS, et diluées une fois dans du DMSO avant agitation 2 minutes (Vortex ou sonification).

### Résultats

Les résultats de solubilité thermodynamique sont donnés dans le tableau 1 ci-dessous :

**Tableau 1 : solubilité thermodynamique élafibranor en milieux aqueux.**

| Solvants | Solubilité après 24 h | | Solubilité après 72 h | |
|---|---|---|---|---|
| | µM | mg/mL | µM | mg/mL |
| Tampon pH 4,6 | 114 | 0,044 | 110 | 0,042 |
| Tampon pH 7,4 | 504 | 0,194 | 609 | 0,234 |
| Tampon pH 8,5 | 4419 | 1,701 | 4270 | 1,644 |
| Propylène Glycol | 33520 | 12,905 | 35081 | 13,506 |
| Polyéthylène Glycol 400 | 39022 | 15,023 | 40971 | 15,774 |

La solubilité de l'élafibranor est faible en milieu aqueux. Elle augmente en fonction du pH, passant de 114 à 4419 µM de pH 4,6 à 8,5. L'ajout de co-solvant comme le propylène glycol ou le PEG 400 permet d'améliorer significativement la solubilité de la molécule.

### EXEMPLE 3: Préparation de sels d'élafibranor (GFT505)

L'un des modes préférés d'administration est la voie parentérale. Afin d'y parvenir, la demanderesse a préparé des sels d'élafibranor dans le but d'améliorer la solubilité de l'élafibranor pour pouvoir réaliser des solutions injectables ou des poudres pour préparation injectable.

### Protocole expérimental

Les sels sont élaborés à partir d'un lot d'élafibranor préalablement synthétisé.

### Sel de choline:

1 g de GFT505 (2,6 mmol) et 104 mg de NaOH (1eq, 2,6mmol) sont mis en suspension dans 10ml d'isopropanol et 14ml de méthanol et chauffés à 65°C. Une solution de 365mg de Choline.Cl (1eq, 2,6mmol) dans 1,5ml d'isopropanol est ajoutée et le milieu réactionnel jaune est agité pendant 30mn à 65°C. Après retour à température ambiante, la fine suspension est filtrée, lavée par 2x1 ml d'isopropanol puis concentrée à sec. Les essais de précipitation/cristallisation du résidu solide jaune n'ont pour le moment pas fonctionné.

Après 5 jours de séchage à 45°C sous vide poussé (<10-2 mbar), on obtient 820mg de solide jaune vif (Lot CP0686). Des traces de solvant (isopropanol 15%) sont détectées par RMN.

Les produits sont conservés au frais (2-8°C) et sous gaz inerte pour éviter toute dégradation. Une analyse complète des sels d'élafibranor est réalisée, incluant identification et pureté chimique.

### Résultats :

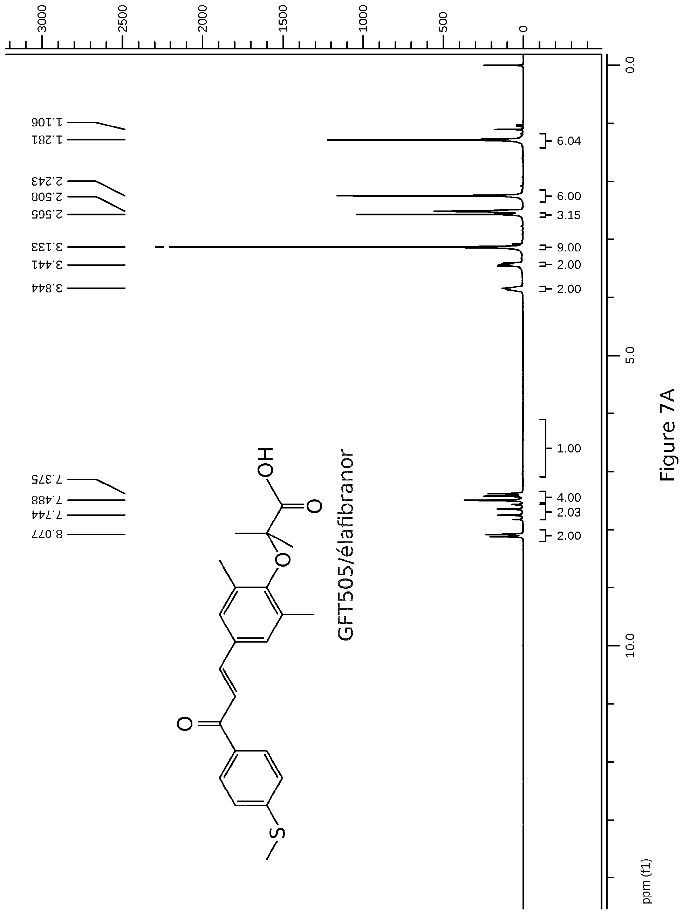

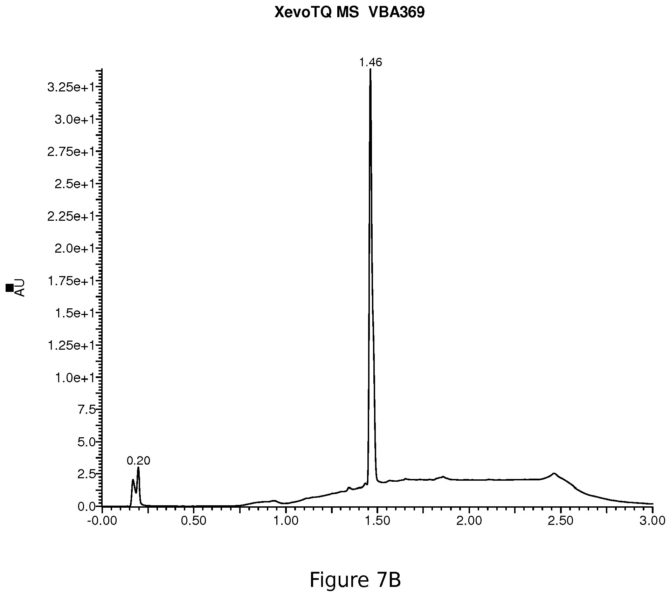

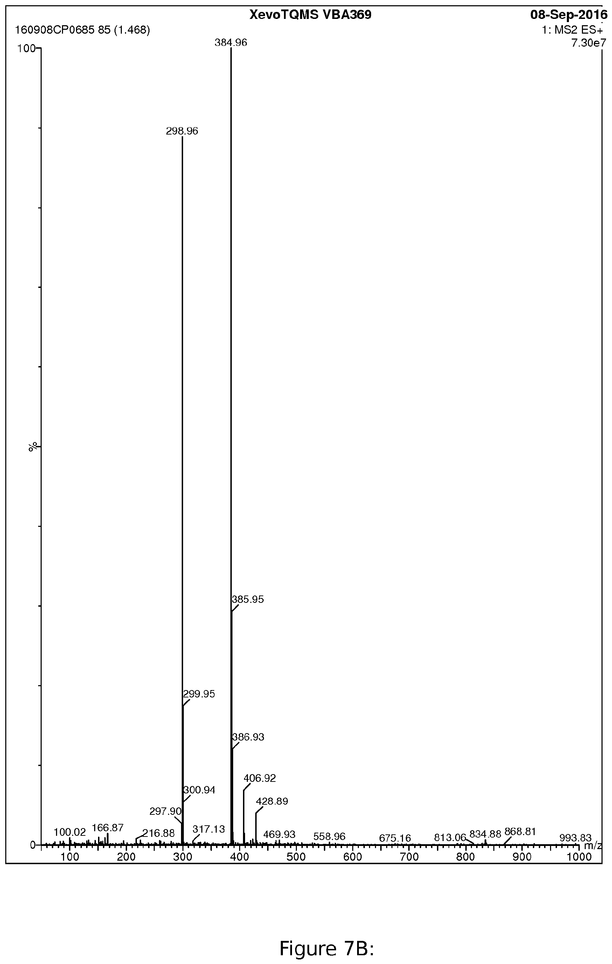

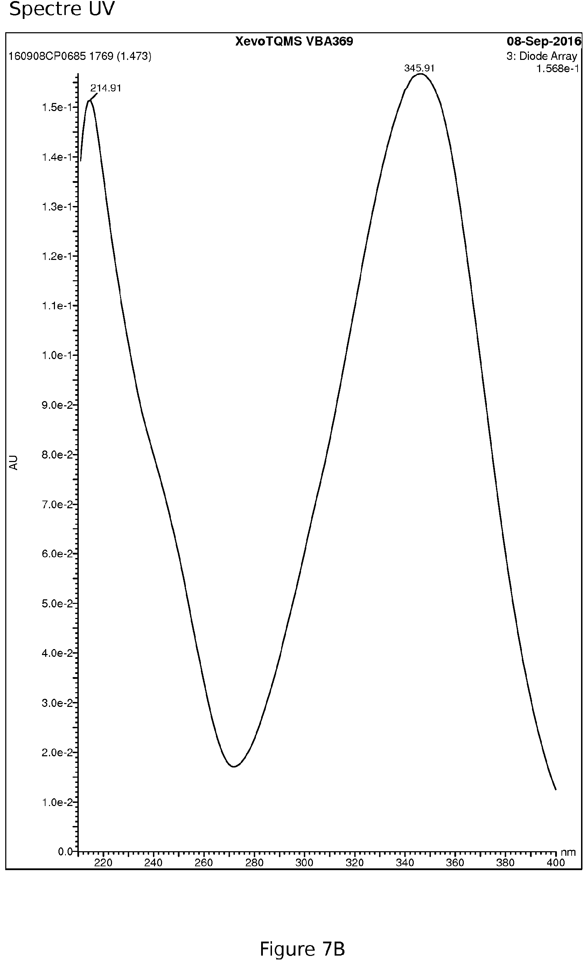

Les résultats sont présentés en figures 7 de manière non exhaustive :

### EXEMPLE 4: Test de solubilité de différents sels d'élafibranor (GFT505)

Cet exemple présente les caractéristiques de solubilité de différentes formes et sels d'élafibranor, en vue d'une administration par voie parentérale ou dans le cadre d'une composition entérale à libération rapide.

La cinétique de solubilisation est déterminée en milieux aqueux (eau et tampons pharmacopées tels pH 7,4 ; 6,0 et 4,5 notamment), à température ambiante. Les résultats sont indiqués dans les tableaux 2 et 3.

**Tableau 2 : solubilité différents sels d'élafibranor.**

| Produits | Poids moléculaire | Point de fusion en °C | Solubilité (selon Ph. Eur.) |
|---|---|---|---|
| élafibranor amorphe | 384 | 144/145 | Très peu soluble |
| Sel d'éthanolamine | 428 | - | Assez soluble |
| Sel de meglumine | 562 | - | Soluble |
| Sel de L-lysine | 513 | - | Très soluble |
| Sel de trométhamine | 488 | - | Soluble |
| Sel de choline | 487 | 197/199 | Très soluble |

**Tableau 3 : solubilité élafibranor base libre et sel de choline en milieux aqueux.**

| Solvants | Elafibranor (GFT505) sel de choline | | Elafibranor (GFT505) Base libre amorphe | |
|---|---|---|---|---|
| | µM | mg/mL | µM | mg/mL |
| Tampon pH 4,6 | ND | ND | 114 | 0,044 |
| Tampon pH 7,4 | 82000 | 40 | 504 | 0,194 |
| Tampon pH 8,5 | ND | ND | 4419 | 1,701 |
| Propylène Glycol | - | - | 33520 | 12,905 |
| Polyéthylène Glycol 400 | - | - | 39022 | 15,023 |

Le sel de choline a une solubilité au moins 150 fois supérieure à l'élafibranor sous sa forme de base libre.

### EXEMPLE 5: Cinétique de dissolution de formulations orales de sels d'élafibranor.

La demanderesse a étudié le profil de dissolution de plusieurs lots de gélules contenant respectivement des sels de choline et de l'élafibranor base libre de structure amorphe, en dose unitaire équivalente à 120 mg sous forme d'élafibranor base.

Les tests de dissolution ont été réalisés sur la plupart des formulations dans des milieux tamponnés à pH 6.0 USP et dans des milieux gastriques simulant les conditions nourries et â jeun, avec apport d'enzymes (milieux dénommés respectivement FeSSIF et FaSSIF). Le système USP type II à palettes a été retenu pour les essais avec 1000 ml de volume, 37°C et les prélèvements, réalisés à 0, 2, 5, 10, 15, 20, 30, 45 and 60 minutes ont été analysés par HPLC sans renouvellement des milieux.

### Résultats

Les résultats montrent que l'élafibranor sous la forme sel de choline est très rapidement dissous dans les deux milieux FaSSIF et FeSSIF. Au terme de l'essai (60 minutes), 100 % de la substance active est dissoute pour les formulations et il n'y a pas de différences selon le milieu de dissolution étudié pour le sel de choline d'élafibranor, ce qui n'est pas le cas pour les formulations contenant l'élafibranor sous forme de base libre.

La cinétique de dissolution est notablement plus rapide pour les formulations à base de sel de choline.

En conclusion, l'impact du sel d'élafibranor sur les propriétés de dissolution sont surprenantes. Les essais sur les formulations à base de sel ont un profil similaire quel que soit le milieu simulant la prise ou non de repas.

Sur la base de ces données *in vitro* prédictives, il est donc probable que les formulations à base de sels de choline aient une absorption améliorée liée à une solubilité supérieure et une dissolution plus rapide, permettant de s'affranchir de tout impact/toute variation liée à la prise d'aliments.

### EXEMPLE 6: Stabilité de l'élafibranor et de ses sels.

La demanderesse a décidé de vérifier la stabilité de l'élafibranor sous forme de base libre et de sels après exposition à la lumière et à température ambiante.

### Protocole expérimental

Des échantillons ont été préparés sous forme de poudre seule et de solutions aqueuses pour les échantillons suivants : élafibranor, élafibranor choline.

La stabilité est mesurée sur une période de 7 à 14 jours par UPLCMS, avec calcul du taux de recouvrement du pic de l'élafibranor par rapport à la valeur initiale et mesure de son indice de pureté. Les produits sont exposés à la lumière du jour et à température ambiante.

Les échantillons références sont quant à eux stockés au frais (2-8°C), protégés de la lumière par un papier aluminium et sous gaz inerte pour le produit solide.

### Résultats

Le tableau 4 ci-après montre, en exemple, les résultats qualitatifs de photostabilité du sel de choline (recouvrement en %) selon son état physique (solution ou poudre) après conservation de deux semaines.

**Tableau 4 : photostabilité élafibranor sels (recouvrement/théorie)**

| Produits | Données T0 | Stabilité 14 jours (solution) | Stabilité 14 jours (poudre) |
|---|---|---|---|
| Sel de choline | 100 | >90 % | ∼90% |

Ils démontrent que l'élafibranor sous forme de sel de choline, a un changement moyen de coloration à la lumière (photosensibilité), jaune à jaune moyen, que ce soit sous forme poudre ou bien en solution, avec une intensité moindre que le GT505 sous sa forme de base libre. Le recouvrement après 14 jours de stockage est compris dans la norme 100 ± 10 %.

La température n'a pas d'impact sur la stabilité. Les produits de dégradation à la lumière n'ont pas été identifiés.

## Revendications

1. Composition comprenant comme principe actif un sel pharmaceutiquement acceptable d'élafibranor **caractérisée en ce que** le sel pharmaceutiquement acceptable d'élafibranor est un sel de choline.

2. Composition selon la revendication précédente **caractérisée en ce qu'**elle est sous une forme adaptée pour une administration par voie entérale.

3. Composition selon la revendication 1 **caractérisée en ce qu'**elle est sous une forme adaptée pour une administration par voie parentérale.

4. Composition selon la revendication précédente **caractérisée en ce qu'**elle est sous une forme adaptée pour une administration par voie intraveineuse.

5. Composition selon l'une quelconque des deux revendications précédentes dans laquelle le sel d'élafibranor est micronisé ou de structure amorphe.

6. Composition selon l'une quelconque des trois revendications précédentes dans laquelle le sel d'élafibranor est sous forme d'une poudre pour préparation injectable soluble.

7. Composition selon la revendication 1 **caractérisée en ce qu'**elle est sous une forme adaptée pour une administration par voie sous-cutanée.

8. Composition selon l'une quelconque des revendications précédentes **caractérisée en ce qu'**elle comporte au moins un excipient choisi parmi les liants, les agents désintégrants, les diluants, les lubrifiants, les agents tensioactifs, les agents tampons, les agents d'écoulement, les colorants, les arômes, les édulcorants, les solvants ou agents conservateurs.

9. Composition selon l'une quelconque des revendications précédentes **caractérisée en ce qu'**elle comporte plus de 50% des particules de taille inférieure ou égale à 10 µm et l'ensemble des particules de taille inférieure à 20 µm.

10. Composition selon l'une quelconque des revendications précédentes **caractérisée en ce que** le sel pharmaceutiquement acceptable d'élafibranor est configuré pour avoir une solubilité au moins égale à 10 mg/ml en milieu physiologique NaCl 0.9 %.

11. Composition selon l'une quelconque des revendications précédentes **caractérisée en ce que** le sel d'élafibranor est configuré pour présenter un profil de dissolution en milieux FaSSIF et FeSSIF simulés supérieur à 90 % après 30 minutes.

12. Composition selon l'une quelconque des revendications précédentes pour laquelle le sel pharmaceutiquement acceptable d'élafibranor (GFT505) est configuré pour être photostable.

13. Composition selon l'une quelconque des revendications précédentes pour son utilisation dans le traitement des maladies hépatiques.

14. Composition pour son utilisation dans le traitement des maladies hépatiques selon la revendication précédente dans laquelle la maladie hépatique consiste en la stéatose hépatique non alcoolique (NAFLD) ou en la stéato-hépatite non alcoolique (NASH), ou en la fibrose hépatique, ou en la cirrhose, ou en des maladies auto-immunes hépatiques.

## Patentansprüche

1. Zusammensetzung, umfassend als Wirkprinzip ein pharmazeutisch akzeptables Elafribranorsalz, **dadurch gekennzeichnet, dass** das pharmazeutisch akzeptable Elafribranorsalz ein Cholinsalz ist.

2. Zusammensetzung nach vorangehendem Anspruch, **dadurch gekennzeichnet, dass** sie in angepasster Form für eine enterale Verabreichung vorliegt.

3. Zusammensetzung nach Anspruch 1, **dadurch gekennzeichnet, dass** sie in angepasster Form für eine parenterale Verabreichung vorliegt.

4. Zusammensetzung nach vorangehendem Anspruch, **dadurch gekennzeichnet, dass** sie in angepasster Form für eine intravenöse Verabreichung vorliegt.

5. Zusammensetzung nach einem der zwei vorangehenden Ansprüche, wobei das Elafibranorsalz mikronisiert oder amorpher Struktur ist.

6. Zusammensetzung nach einem der drei vorangehenden Ansprüche, wobei das Elafribranorsalz in Pulverform für ein lösliches injizierbares Präparat vorliegt.

7. Zusammensetzung nach Anspruch 1, **dadurch gekennzeichnet, dass** sie in angepasster Form für eine subkutane Verabreichung vorliegt.

8. Zusammensetzung nach einem der vorangehenden Ansprüche, **dadurch gekennzeichnet, dass** sie mindestens einen Hilfsstoff aufweist, der aus den Bindemitteln, den Zerfallsmitteln, den Verdünnern, den Schmiermitteln, den Tensiden, den Puffermitteln, den Fließmitteln, den Farbstoffen, den Aromen, den Süßungsmitteln, den Lösungsmitteln oder den Konservierungsstoffen ausgewählt ist.

9. Zusammensetzung nach einem der vorangehenden Ansprüche, **dadurch gekennzeichnet, dass** sie über 50 % Partikel mit einer Größe von kleiner oder gleich 10 µm und die Gesamtheit der Partikel mit einer Größe von unter 20 µm aufweist.

10. Zusammensetzung nach einem der vorangehenden Ansprüche, **dadurch gekennzeichnet, dass** das pharmazeutisch akzeptable Elafribranorsalz ausgelegt ist, um eine Löslichkeit von mindestens gleich 10 mg/ml in physiologischem Milieu NaCl 0,9 % zu haben.

11. Zusammensetzung nach einem der vorangehenden Ansprüche, **dadurch gekennzeichnet, dass** das Elafribranorsalz ausgelegt ist, um ein Auflösungsprofil in simulierten FaSSIF- und FeSSIF-Milieus von über 90 % nach 30 Minuten zu haben.

12. Zusammensetzung nach einem der vorangehenden Ansprüche, für die das pharmazeutisch akzeptable Elafribranorsalz (GFT505) ausgelegt ist, um photostabil zu sein.

13. Zusammensetzung nach einem der vorangehenden Ansprüche für ihre Verwendung bei der Behandlung von Lebererkrankungen.

14. Zusammensetzung für ihre Verwendung bei der Behandlung von Leberkrankungen nach vorangehendem Anspruch, wobei die Lebererkrankung aus der nichtalkoholischen Leberstenose (NAFLD) oder aus der nichtalkoholischen Steatohepatitis (NASH) oder aus der Leberfibrose oder aus der Zirrhose oder aus den Autoimmun-Lebererkrankungen besteht.

## Claims

1. Composition comprising as an active ingredient a pharmaceutically acceptable salt of elafibranor **characterised in that** the pharmaceutically acceptable salt of elafibranor is a choline salt.

2. Composition according to the preceding claim **characterised in that** it is in a form adapted for administration by the enteral route.

3. Composition according to claim 1 **characterised in that** it is in a form adapted for administration by the parenteral route.

4. Composition according to the preceding claim **characterised in that** it is in a form adapted for administration by the intravenous route.

5. Composition according to any one of the two preceding claims wherein the elafibranor salt is micronised or of amorphous structure.

6. Composition according to any one of the three preceding claims wherein the salt of elafibranor is in the form of a powder for soluble injectable preparation.

7. Composition according to claim 1 **characterised in that** it is in a form adapted for administration by the subcutaneous route.

8. Composition according to any one of the preceding claims **characterised in that** it includes at least one excipient selected from binders, disintegrating agents, diluents, lubricants, surfactants, buffer agents, flow agents, colorants, flavours, sweeteners, solvents or preservatives.

9. Composition according to any one of the preceding claims **characterised in that** it includes more than 50% particles less than or equal to 10 µm in size and all particles less than 20 µm in size.

10. Composition according to any one of the preceding claims **characterised in that** the pharmaceutically acceptable salt of elafibranor is configured to have a solubility at least equal to 10 mg/ml in 0.9% NaCl saline solution.

11. Composition according to any one of the preceding claims **characterised in that** the salt of elafibranor is configured to have a dissolution profile in simulated FaSSIF and FeSSIF media greater than 90% after 30 minutes.

12. Composition according to any one of the preceding claims for which the pharmaceutically acceptable salt of elafibranor (GFT505) is configured to be photostable.

13. Composition according to any one of the preceding claims for use thereof in the treatment of liver diseases.

14. Composition for use thereof in the treatment of liver diseases according to the preceding claim wherein the liver disease consists of non-alcoholic fatty liver disease (NAFLD) or non-alcoholic steatohepatitis (NASH), or liver fibrosis, or cirrhosis, or autoimmune liver diseases.
